# EUROPÄISCHE PATENTSCHRIFT

(11) **EP 3 773 786 B1**
(45) Veröffentlichungstag und Bekanntmachung des Hinweises auf die Patenterteilung: **22.01.2025**
(21) Anmeldenummer: 19716318.1
(22) Anmeldetag: 26.03.2019
(51) Int. Cl.: A61M 1/16

(54) **DIALYSEGERÄT**
DIALYSIS MACHINE
APPAREIL DE DIALYSE

(30) Priorität: 29.03.2018 DE 102018107627
(43) Veröffentlichungstag der Anmeldung: 17.02.2021
(73) Patentinhaber: Fresenius Medical Care Deutschland GmbH, 61352 Bad Homburg (DE)
(72) Erfinder: IRRGANG, Tobias, 97633 Aubstadt (DE); STÄBLEIN, Tilman, 97072 Würzburg (DE); GAGEL, Alfred, 96123 Litzendorf (DE); KLÖFFEL, Peter, 97720 Nüdlingen (DE)
(74) Vertreter: Herrmann, Uwe
(86) Internationale Anmeldenummer: PCT/EP2019/057609
(87) Internationale Veröffentlichungsnummer: WO 2019/185638

(56) Entgegenhaltungen:
- WO-A1-2018/086739
- WO-A1-2018/208498
- WO-A1-93/09822
- WO-A1-94/11093
- WO-A2-2009/015882
- US-A- 5 295 505
- US-A- 5 318 750
- US-A1- 2002 088 752

## Beschreibung

Die Erfindung betrifft ein Dialysegerät, das ausgebildet ist, mit einem extrakorporalen Blutsystem ausgerüstet zu werden, mit einem Dialyselösungssystem, das ausgebildet ist, mit einem Dialysator ausgerüstet zu werden, wobei ein Mischbereich, insbesondere eine Mischkammer, vorgesehen ist, in dem sich im Betrieb des Dialysegerätes die frische Dialyselösung oder ein Bestandteil von dieser befindet, wobei mit dem Mischbereich eine Konzentratleitung in Fluidverbindung steht, durch die Konzentrat aus einem Konzentratbehälter in den Mischbereich einleitbar ist.

Dialysegeräte der eingangs genannten Art sind aus dem Stand der Technik bekannt. Sie werden im Rahmen einer Dialysebehandlung dazu verwendet, Harnstoff und andere Stoffe aus dem Blut eines Patienten mit fehlender oder verminderter Nierenaktivität zu entfernen. Der Dialysator eines Dialysegeräts ist eine Filtereinheit mit einer Blutkammer und einer Dialyselösungskammer, die durch eine semipermeable Membran voneinander getrennt sind. Die aus dem Blut zu entfernenden Stoffe treten im Dialysator durch die semipermeable Membran von der Blutkammer in die Dialyselösungskammer über.

Zum Zwecke der Bilanzierung der dem Dialysator zugeführten und der von diesem abgeführten Menge an Dialyselösung wird üblicherweise eine sogenannte Bilanzkammer eingesetzt. Diese weist zwei durch eine bewegliche Wand voneinander getrennte Bilanzkammerhälften auf, die jeweils über einen Zulauf und über einen Ablauf verfügen, die über je ein Ventil geöffnet und geschlossen werden können. Während eine der Bilanzkammerhälften durch frische Dialyselösung gefüllt wird, bewegt sich die Wand von der sich füllenden Bilanzkammerhälfte weg und verdrängt auf diese Weise die in der anderen Bilanzkammerhälfte befindliche gebrauchte Dialyselösung. Da sich beide Bilanzkammerhälften in derselben Bilanzkammer mit festen Wandungen befinden, entspricht das zugeführte Volumen an frischer Dialyselösung dem abgeführten Volumen an verbrauchter Dialyselösung.

Es wird bereits an dieser Stelle darauf hingewiesen, dass das Dialysegerät gemäß der vorliegenden Erfindung ein derartiges Bilanzkammersystem aufweisen kann, aber nicht zwingenderweise aufweisen muss, es handelt sich somit um ein bevorzugtes, gleichwohl aber optionales Merkmal. Die wenigstens eine Bilanzkammer ist somit ein optionaler Bestandteil des Dialysegerätes. Alternativ zum Einsatz einer oder mehrerer Bilanzkammern ist beispielsweise die Verwendung von Flusssensoren mit Pumpen für das Bilanzieren möglich, was z.B. ebenfalls von der Erfindung umfasst ist.

Grundsätzlich besteht die Möglichkeit, Dialysegeräte mit einer gebrauchsfertigen Dialyselösung zu versorgen, die z.B. in einem Behältnis oder in einer Versorgungsleitung bereitgestellt wird.

Die US5318750 offenbart eine Vorrichtung zur Herstellung einer Dialyselösung durch Auflösen mehrerer pulverförmiger Substanzen in Wasser. Die Vorrichtung umfasst eine Anzahl unabhängiger Kartuschen, die jeweils ein Salz einer für die Herstellung des Dialysats erforderlichen Substanz, wie Natriumchlorid, Kaliumchlorid, Calciumchlorid, Magnesiumchlorid und Natriumbicarbonat aufweisen. Die Vorrichtung umfasst mindestens eine erste Leitung, die mit einem Einlass zu den Kartuschen in Verbindung steht, um Wasser in die Kartuschen einzuführen, um wässrige Lösungen in den Kartuschen zu erzeugen, mindestens eine zweite Leitung die mit einem Auslass aus den Kartuschen in Verbindung steht, um die wässrigen Lösungen zu einem Mischpunkt zu bringen, der sich stromaufwärts von einem Dialysekreislauf befindet stromaufwärts von einem Dialysekreislauf gelegenen Mischpunkt zu bringen, eine erste Messvorrichtung, die an der zweiten Leitung stromaufwärts von dem Mischpunkt angebracht ist, um die Konzentration der wässrigen Lösungen am Auslass der Kartuschen, eine zweite Messvorrichtung, die stromabwärts vom Dialysekreislauf stromabwärts des Dialysekreislaufs montierte zweite Messvorrichtung und mindestens eine Durchflussregulierungsvorrichtung zur Regulierung der Durchflussmenge des Wassers, um die Konzentration der wässrigen Lösungen in Abhängigkeit von den von der ersten und zweiten Messvorrichtung gelieferten Informationen zu verändern.

Aus der US5295505 ist eine Vorrichtung zur Herstellung einer medizinischen Lösung, insbesondere einer Dialyselösung, aus pulverförmigem Konzentrat und Wasser bekannt. Die Vorrichtung umfasst eine Wasserquelle, von der eine erste Leitung zu einem das Pulver enthaltenden Behälter führt und von der eine zweite Leitung, in die eine erste Messzelle und eine Konzentratpumpe geschaltet sind, zu einem Mischpunkt. Diese Mischstelle befindet sich in einer dritten Leitung, die von der Wasserquelle zu einem Verbraucher führt; stromabwärts von dem Mischpunkt ist eine zweite Messzelle zur Überwachung der Zusammensetzung der fertigen Dialyselösung vorgesehen.

Aus dem Stand der Technik ist es ebenso bekannt, die gebrauchsfertige Dialyselösung am Dialysegerät selbst herzustellen, wobei üblicherweise mehrere Konzentrate mit RO-Wasser in einem Mischbereich gemischt werden. Diese Konzentrate werden aus Konzentratbehältern mittels Konzentratpumpen zu dem Mischbereich gefördert, der vorzugsweise als Mischkammer ausgeführt ist. Dabei werden üblicherweise zwei Konzentratbehälter verwendet, von denen einer ein basisches und einer ein saures Konzentrat enthält. Die auf diese Weise erhaltene Mischung stellt die gebrauchsfertige Dialyselösung dar und wird sodann zu der Bilanzkammer gefördert und von dort aus zum Dialysator geleitet.

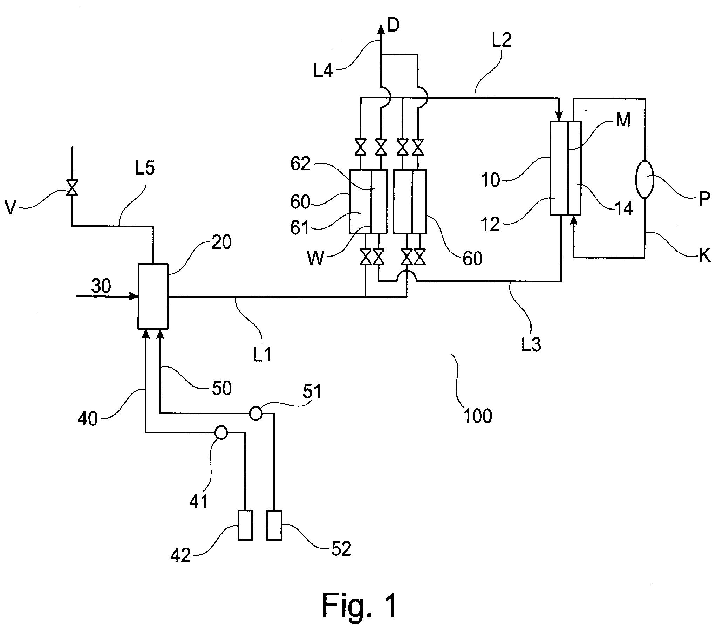

Ein bekanntes Dialysegerät mit Merkmalen des Oberbegriffs des Anspruchs 1 ist in Figur 1 schematisch dargestellt.

Der Patient P steht über den extrakorporalen Blutkreislauf K mit dem Dialysator 10 in Verbindung, der ein blutseitiges 14 und ein dialysatseitiges Kompartiment 12 aufweist, die durch eine semipermeable Membran M voneinander getrennt sind. Das Blut des Patienten wird vorzugsweise im Gegenstrom zu der Dialyselösung durch den Dialysator 10 geleitet, wie dies durch Pfeile angedeutet ist, die die Strömungsrichtung wiedergeben.

Das hydraulische System des Dialysegerätes, d.h. das Dialyselösungssystem, ist allgemein mit dem Bezugszeichen 100 gekennzeichnet.

Die Dialyselösung wird in der Mischkammer 20 aus der durch die Leitung 30 einströmenden Flüssigkeit, vorzugsweise RO-Wasser (RO = Reversosmose) sowie aus den beiden Konzentraten hergestellt, die durch die Leitungen 40, 50 in die Mischkammer 20 einströmen. Die Förderung der Konzentrate erfolgt mittels der Pumpen 41, 51, die das Konzentrat aus den Konzentratbehältern 42, 52 fördern.

Die an der Mischkammer 20 angeordnete Leitung L5 dient zur Entlüftung der Dialyselösung und ist durch das Ventil V verschließbar.

Die Bilanzkammer 60 weist die oben genannten Bilanzkammerhälften 61 und 62 auf, die durch die bewegliche, nicht für Dialyselösung permeable Wand W voneinander getrennt sind.

Um einen kontinuierlichen Fluss zu gewährleisten, sind zwei parallel geschaltete Bilanzkammern 60 vorgesehen, wie dies aus Figur 1 hervorgeht. Diese können identisch aufgebaut sein. Die Bilanzkammern sind insofern vereinfacht dargestellt, als dass deren Innenwandungen vorzugsweise so ausgeformt sind, dass sich die Wand W in ihren Extrempositionen vollständig an diese anlegen kann.

Jede Bilanzkammerhälfte 61, 62 einer Bilanzkammer 60 weist je ein Ventil auf der Ein- und auf der Auslassseite auf, um den Zu- und Ablauf in die und aus den Bilanzkammerhälften zu steuern.

Die beiden links dargestellten Bilanzkammerhälften 61 der Bilanzkammern 60 dienen zur Aufnahme frischer Dialyselösung aus der Leitung L1. Aus den Bilanzkammerhälften 61 gelangt die Dialyselösung über die Leitung L2 in das dialysatseitige Kompartiment 12 des Dialysators 10. Die beiden rechts dargestellten Bilanzkammerhälften 62 der Bilanzkammern 60 dienen zur Aufnahme verbrauchter Dialyselösung, die über die Leitung L3 von dem Dialysator 10 zu den Bilanzkammerhälften 62 gelangt und nach der Bilanzkammer über die Leitung L4 zu einem Ablauf (Drain D) geführt wird.

Ist eines oder sind beide der Konzentratbehältnisse 42, 52 entleert, kann der Fall eintreten, dass Luft in die Bilanzkammer oder in ein sonstiges Bilanziersystem gelangt, was zu einem Bilanzierfehler führt, da die betroffene Bilanzkammerhälfte der Bilanzkammer oder eine Pumpe zur bilanzierenden Förderung der Lösung dann nicht vollständig mit frischer Dialyselösung befüllt wird, sondern teilweise mit Luft, und somit weniger frische Dialyselösung zum Dialysator gefördert wird, als angenommen. In diesen Fällen wird Luft über die Konzentratleitungen in die Mischkammer und von dort aus in die Bilanzkammer oder ein sonstiges Bilanziersystem gefördert bzw. mitgerissen.

Dieses Mitreißen der Luft erfolgt z.B. durch das Öffnen des Entlüftungsventils V zur Totzeit der Bilanzkammer. Unter der Totzeit der Bilanzkammer ist die Zeitspanne zu verstehen, in der sich die Wand W in einer Extremlage (Auslenkung ganz nach rechts oder links) befindet und alle Ventile der Bilanzkammer geschlossen sind. Dieser Zustand kann beispielsweise 100 ms andauern.

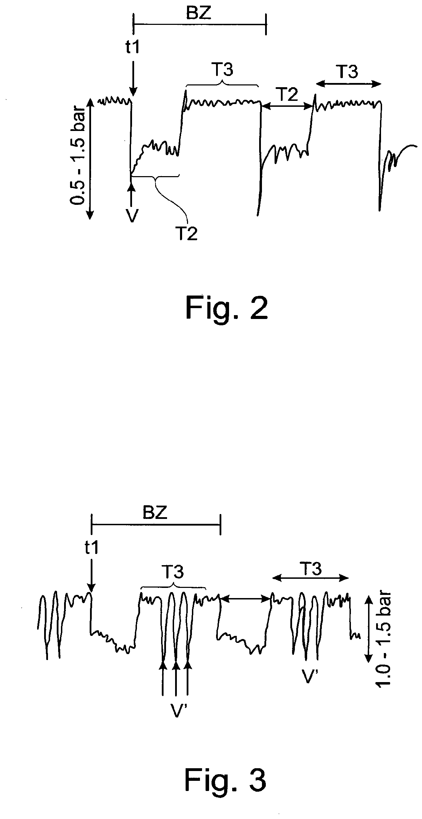

Figur 2 zeigt den zeitlichen Druckverlauf während eines Bilanzkammerzyklus, wobei der Zeitpunkt t1 die genannte Totzeit kennzeichnet. Wie aus Figur 2 durch einen Pfeil V gekennzeichnet, wird zu diesem Zeitpunkt t1 das Ventil V kurzzeitig geöffnet. Anschließend wird das Ventil V geschlossen und das Zulaufventil der Bilanzkammer geöffnet, so dass der Druck in der sich füllenden Bilanzkammerhälfte 61 ansteigt. Diese Füllphase ist in Figur 2 mit T2 gekennzeichnet. In dem sich daran anschließenden Zustand T3 ist die Bilanzkammerhälfte vollständig gefüllt, was auch als Hochdruckphase der Bilanzkammer bezeichnet wird.

Das Bezugszeichen BZ kennzeichnet den gesamten Bilanzkammerhälftenzyklus, der alle diese Phasen bzw. Zeitpunkte umfasst.

Die Luft steigt bei offenem Ventil V in den Leitungen 40, 50 nach oben und wird dann durch den Füllfluss, mittels dessen die Bilanzkammer 61 gefüllt wird, in die Bilanzkammer bzw. Bilanzkammerhälfte 61 mitgerissen. Dies führt zu einer Fehlbilanz.

Der vorliegenden Erfindung liegt daher die Aufgabe zugrunde, ein Dialysegerät gemäß dem Oberbegriff des Anspruchs 1 dahingehend weiterzubilden, dass die Wahrscheinlichkeit für einen Lufteintrag in die Dialyselösung bzw. in ein bilanzierendes System des Dialysegerätes gegenüber bekannten Dialysegeräten verringert wird.

Diese Aufgabe wird durch ein Dialysegerät mit den Merkmalen des Anspruchs 1 gelöst.

Danach ist vorgesehen, dass in oder an der oder den Konzentratleitungen voneinander beabstandet zumindest zwei elektrisch leitfähige Elemente angeordnet sind, dass eine Messeinrichtung vorgesehen ist, die mit den leitfähigen Elementen in elektrischer Verbindung steht und die ausgebildet ist, zwischen diesen den Leitwert oder eine damit korrelierte Größe zu messen, und dass eine Steuereinheit vorgesehen ist, die ausgebildet ist, den gemessenen Wert mit einem Sollwert oder mit einem Grenzwert oder mit einem Sollwertbereich zu vergleichen.

Dabei können die wenigstens zwei leitfähigen Elemente in oder an derselben Konzentratleitung und/oder in oder an verschiedenen Konzentratleitungen angeordnet sein.

Denkbar ist es, dass in oder an einer Konzentratleitung jeweils genau ein elektrisch leitfähiges Element angeordnet ist und/oder dass eine oder mehrere Konzentratleitungen vorgesehen sind, in oder an der jeweils mehrere elektrisch leitfähige Elemente angeordnet sind. Denkbar ist somit eine Ausführungsform, in der eine (oder mehrere) Konzentratleitungen jeweils mit zumindest zwei elektrisch leitfähigen Elementen versehen ist/sind. Alternativ oder zusätzlich kann vorgesehen sein, dass zwei oder n (n >2) Konzentratleitungen mit insgesamt zwei oder n (n>2) elektrisch leitfähigen Elementen vorliegen, also je Konzentratleitung ein elektrisch leitfähiges Element vorgesehen ist.

Die Messung findet zwischen den zwei elektrisch leitfähigen Elementen statt, unabhängig davon, ob sich diese in ein und derselben oder in unterschiedlichen Konzentratleitungen befinden.

Das oder die elektrisch leitfähigen Elemente können als Elektroden ausgeführt sein.

Denkbar ist eine Anordnung, bei der in zwei Konzentratleitungen jeweils ein oder mindestens ein elektrisch leitfähiges Element angeordnet ist und dass die zwei Konzentratleitungen zusammen mit der Mischkammer oder einem sonstigen Mischbereich eine leitfähige Strecke ausbilden, über die ein Signal (z.B. der Leitwert oder eine damit korrelierte Größe) von dem elektrisch leitfähigen Element einer Konzentratleitung zu dem elektrisch leitfähigen Element der anderen Konzentratleitung gemessen werden kann. Bei einer Konzentratleitung kann es sich beispielsweise um die Leitung für das saure Konzentrat und bei der anderen Konzentratleitung um die Leitung für das basische Konzentrat handeln. Dieses Signal wird unterbrochen, wenn einer oder beide Konzentratbehälter leer sind bzw. Luft oder ein Luft/Konzentrat-Gemisch durch die Konzentratleitungen gefördert wird.

Die Konzentratleitungen sind vorzugsweise als Schläuche ausgeführt.

Somit wird der Leitwert, d.h. der Kehrwert des ohmschen Widerstands oder eine damit korrelierte Größe, wie z.B. die Spannung, die Stromstärke, der ohmsche Widerstand etc. gemessen, wobei die Messung zwischen den zwei elektrisch leitfähigen Elementen stattfindet.

Befindet sich Konzentrat in der Konzentratleitung, d.h. in der Zuführung, ist der elektrische Kreis geschlossen und es ergibt sich ein hoher Leitwert. Kommt Luft durch die Konzentratleitung, sinkt der Leitwert. Unterschreitet der Leitwert einen Grenzwert, d.h. bewegt sich dieser aus einem Sollwertbereich heraus, kann auf das Vorliegen von Luft erkannt werden und beispielsweise Alarm ausgelöst werden.

Die elektrisch leitfähigen Elemente können beispielsweise durch Schlauchtüllen gebildet werden, die in Längsrichtung der Konzentratleitung voneinander beabstandet angeordnet sind.

Die elektrisch leitfähigen Elemente sind an oder in der oder den Konzentratleitungen so angeordnet, dass diese mit dem Inhalt der Konzentratleitung, d.h. mit dem Konzentrat, Luft etc. in Kontakt kommen.

Die elektrisch leitfähigen Elemente bestehen vorzugsweise aus Metall, vorzugsweise aus Edelstahl und/oder Titan.

Vorzugsweise befindet sich in bzw. an der Konzentratleitung eine Konzentratpumpe, wobei sich die leitfähigen Elemente in Strömungsrichtung des Konzentrats vorzugsweise stromaufwärts der Konzentratpumpe befinden, um eine verschlechterte Erkennung von Luft, wie sie stromabwärts der Konzentratpumpe vorliegt, zu vermeiden. Grundsätzlich ist von der Erfindung auch der Fall umfasst, dass sich die leitfähigen Elemente in Strömungsrichtung des Konzentrats stromabwärts der Konzentratpumpe befinden.

Die Steuereinheit ist ausgebildet, eine besondere Betriebsweise des Dialysegerätes und ggf. einen Alarm zu initiieren, wenn der gemessene Wert (Leitwert oder ein damit korrelierter Wert) nicht mit dem Sollwert übereinstimmt, einen Grenzwert über- oder unterschreitet oder nicht in einem Sollwertbereich liegt. Dies ist wie oben ausgeführt insbesondere dann der Fall, wenn sich in dem Bereich der Konzentratleitung zwischen den elektrisch leitfähigen Elementen Luft oder ein Luft/Konzentratgemisch und kein reines Konzentrat befindet.

Vorzugsweise ist die Steuereinheit bzw. deren Erkennungslogik auf einen bestimmten Abstand der beiden leitfähigen Elemente voneinander ausgelegt. Wird der Abstand zwischen den leitfähigen Elementen verändert, kann es erforderlich sein, auch die Erkennungslogik der Steuereinheit entsprechend anzupassen.

In einer weiteren Ausführung der Erfindung ist die Steuereinheit derart ausgebildet, dass der Alarm und/oder die besondere Betriebsweise des Dialysegerätes erst dann initiiert wird, wenn der gemessene Wert häufiger als ein Schwellenwert nicht mit dem Sollwert übereinstimmt oder einen Grenzwert über- oder unterschreitet oder nicht in einem Sollwertbereich liegt.

Weist das Dialysegerät ein Bilanzkammersytem auf, ist es denkbar, dass ein Zähler um den Wert eins erhöht wird, wenn bei einem Bilanzkammerzyklus Luft in der Konzentratleitung erkannt wird, und um den Wert eins verringert wird, wenn bei einem Bilanzkammerzyklus keine Luft in der Konzentratleitung erkannt wird. Wird auf diese Weise beispielsweise der Wert 5 (oder ein anderer Wert) überschritten oder erreicht, wird ein Alarm ausgelöst und/oder die besondere Betriebsweise des Dialysegerätes initiiert. Diese Vorgehensweise verhindert, dass kleine Luftblasen oder unvorhergesehene Sensorfluktuationen jedes Mal einen Alarm auslösen und/oder die besondere Betriebsweise des Dialysegerätes initiieren.

Dieses System ist nicht auf das Vorhandensein eines Bilanzkammersystems beschränkt, sondern umfasst auch Systeme ohne Bilanzkammersystem.

Die besondere Betriebsweise kann eine vom Normalbetrieb abweichende Spezialluftabscheidung und/oder die Spülung des Dialyselösungssystems oder eines Teils von diesem und/oder die Erhöhung der Flussrate der Dialyselösung und/oder die Bypassschaltung des Dialysators und ggf. weitere Maßnahmen umfassen.

Die vorliegende Erfindung betrifft ein Dialysegerät, das zur Aufnahme eines extrakorporalen Blutsystems, wie z.B. eines Blutschlauchsets und das zur Aufnahme eines Dialysators ausgerüstet ist. Das extrakorporale Blutsystem und/oder der Dialysator sind somit keine zwingenden Bestandteile des Gerätes. Gleichwohl umfasst die Erfindung auch ein Dialysegerät, bei dem das extrakorporale Blutsystem und/oder der Dialysator in das Dialysegerät eingesetzt sind.

Unter einer Bypassschaltung des Dialysators ist zu verstehen, dass die Dialyselösung nicht durch den Dialysator strömt, sondern in einer Bypassleitung an dem Dialysator vorbeigeleitet wird.

Die Spülung des Dialyselösungssystems oder eines Teils von diesem erfolgt erst nach der Spezialluftabscheidung. Die Spülung stellt sicher, dass in dem Dialyselösungskreislauf gebrauchsfertige Dialyselösung mit der korrekten Zusammensetzung vorliegt, wenn der Patient wieder angeschlossen und die Behandlung fortgesetzt wird.

Vorzugsweise ist vorgesehen, dass sich in der Konzentratleitung und/oder zwischen der Mündung der Konzentratleitung in den Mischbereich und einer Bilanzkammer oder eines sonstigen Bilanziersystems des Dialyselösungssystems kein Luftabscheidemittel, insbesondere keine Luftabscheidekammer befindet.

Dadurch lässt sich eine Kostenreduktion erzielen. Vorzugsweise ist vorgesehen, dass die Steuereinheit die Konzentratpumpe weiter betreibt, so dass (nach dem Anschluss eines neuen Konzentratbehälters) gewartet werden muss, bis das Konzentrat durch die normale Pumpenaktivität angesaugt wird. Um die bis dahin angesaugte Luft und die fehlerhaft gemischte Dialyselösung aus dem Dialyselösungssystem zu entfernen, werden vorzugsweise die Spezialluftabscheidung und das Spülen des Dialyselösungssystems, d.h. der Hydraulik oder eines Teils von dieser durchgeführt.

Vorzugsweise ist die Steuereinheit derart ausgebildet, dass diese in dem Betriebszustand der Spezialluftabscheidung des Dialysegerätes ein Ventil ein- oder mehrmals öffnet und schließt, wenn sich die mit der genannten Leitung in Fluidverbindung stehende Bilanzkammerhälfte der Bilanzkammer in ihrer Hochdruckphase befindet. Dabei sind sämtliche Ventile der Bilanzkammer geschlossen. Durch diese Spezialluftabscheidung wird Luft aus dem Mischbereich, aus der oder den Konzentratleitungen und aus der zu der Bilanzkammer führenden Leitung entfernt.

Der Mischbereich, insbesondere die Mischkammer kann mit einem Frischwasseranschluss, insbesondere mit einem RO-Wasseranschluss in Verbindung stehen oder mit diesem verbindbar sein.

Ein Verfahren zum Berteiben eines Dialysegerätes nach einem der vorhergehenden Ansprüche kann so ausgebildet sein, dass an zwei elektrisch leitfähigen und voneinander beabstandeten Elementen in oder an der oder den Konzentratleitungen der Leitwert oder eine damit korrelierte Größe gemessen wird wobei der gemessene Wert mit einem Sollwert, Grenzwert oder mit einem Sollwertbereich verglichen wird. Zu der Anordnung der elektrisch leitfähigen Elemente gelten die obigen Ausführungen zum Dialysegerät entsprechend.

Die Steuereinheit kann durch ein einziges Bauteil gebildet werden, das verschiedene Aufgaben übernimmt, wie z.B. den Vergleich des gemessenen Wertes mit einem Sollwert, Grenzwert oder Sollwertbereich, das Initiieren des Alarms und/oder der besonderen Betriebsweise etc. Die Steuereinheit kann aber auch durch mehrere gesonderte Bauteile gebildet werden, die verschiedene dieser Aufgaben übernehmen, d.h. die Steuereinheit muss nicht zwingend aus einem einzigen Bauteil bestehen.

Vorzugsweise ist vorgesehen, dass wenn der gemessene Wert nicht mit einem Sollwert übereinstimmt oder nicht im einem Sollwertbereich liegt oder einen Grenzwert über- oder unterschreitet, ein Alarm und/oder eine besondere Betriebsweise des Dialysegerätes initiiert wird.

Wie oben ausgeführt, kann die besondere Betriebsweise eine vom Normalbetrieb abweichende Spezialluftabscheidung und/oder die Spülung des Dialyselösungssystems oder eines Teils von diesem und/oder die Erhöhung der Flussrate der Dialyselösung und/oder die Bypassschaltung des Dialysators umfassen.

Denkbar ist es, dass in dem Betriebszustand der Spezialluftabscheidung des Dialysegerätes das Ventil ein- oder mehrmals geöffnet und geschlossen wird, wenn sich die mit der Leitung in Fluidverbindung stehende Bilanzkammerhälfte der Bilanzkammer in ihrer Hochdruckphase befindet, um aus dem Mischbereich sowie aus dem damit verbundenen Leitungssystem Luft zu entfernen. Durch das vorzugsweise mehrfache, beispielsweise dreifache Öffnen und Schließen des Ventils wir die Luft mechanisch ausgetrieben, so dass weniger Restluft in der Mischkammer verbleibt, die bei der nächsten Füllung der Bilanzkammer mitgerissen werden könnte.

Weiterhin kann vorgesehen sein, dass der Betriebszustand der Spezialluftabscheidung nicht gewählt wird, wenn der gemessene Wert einem Sollwert entspricht oder in einem Sollwertbereich liegt bzw. einen bestimmten Grenzwert nicht über- oder unterschreitet und dass stattdessen in diesem Fall eine normale Luftabscheidung vorgenommen wird, bei der das Ventil vorzugsweise nur in der Totzeit der Bilzanzkammer geöffnet wird.

Vorzugsweise steht die Mischkammer oder ein sonstiger Mischbereich mit einer oder mehreren Konzentratleitungen in Verbindung, durch die Konzentrate aus Konzentratbehältern in die Mischkammer gelangen. Bei diesen Konzentraten kann es sich beispielsweise um ein saures und um ein basisches Konzentrat handeln. Üblicherweise steht die Mischkammer mit einem Frischwasseranschluss in Verbindung, insbesondere mit einem RO-Wasseranschluss, so dass das oder die Konzentrate auf das gewünschte Niveau verdünnt werden können. Es wird Bezug genommen auf das Ausführungsbeispiel gemäß Figur 1, das ein nicht beschränkendes Beispiel für ein Dialysegerät der vorliegenden Erfindung darstellt, wobei in Figur 1 nicht die leitfähigen Elemente in den Konzentratleitungen und nicht die Steuereinheit gezeigt sind.

Die vorliegende Erfindung kommt vorzugsweise bei Dialysegeräten zum Einsatz, die über keine Luftabscheidevorrichtung verfügen.

An dieser Stelle wird darauf hingewiesen, dass die Verwendung des Begriffs "ein" oder "eine" auch aber nicht zwingend bedeutet, dass genau eines der fraglichen Elemente vorgesehen ist, sondern auch die Mehrzahl von Elementen mit einschließt. Ebenso ist darauf hinzuweisen, dass die Verwendung der Mehrzahl grundsätzlich auch eines der fraglichen Elemente mit einschließt.

Weitere Einzelheiten und Vorteile der Erfindung werden anhand eines in der Zeichnung dargestellten Ausführungsbeispiels näher erläutert. Es zeigen:
- Figur 1:: eine vereinfachte schematische Darstellung des Hydraulik- und Blutkreislaufes eines Dialysegerätes;
- Figur 2:: den Verlauf des Druckes über die Zeit bei einer normalen Luftabscheidung,
- Figur 3:: den Verlauf des Druckes über die Zeit bei einer Spezialluftabscheidung und
- Figur 4:: eine vereinfachte schematische Darstellung zweier Konzentratleitungen eines Dialysegerätes gemäß der Erfindung.

Figur 1 zeigt einen hydraulischen Kreislauf sowie auch den extrakorporalen Blutkreislauf eines aus dem Stand der Technik bekannten Dialysegerätes. Dieser Aufbau kann identisch oder auch in abgewandelter Form auch bei einem Dialysegerät gemäß der Erfindung vorliegen, so dass die obigen Ausführungen auch für die vorliegende Erfindung gelten.

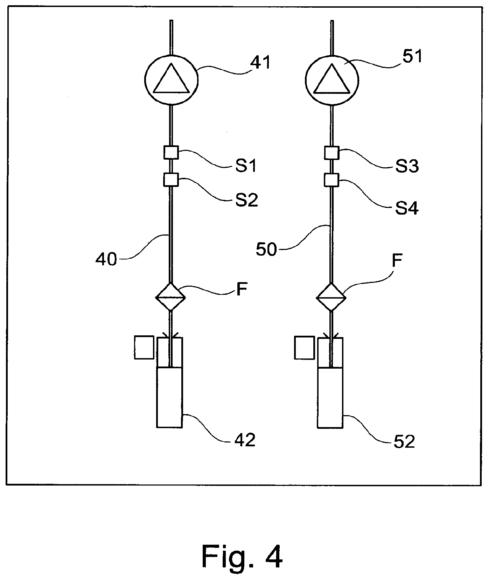

Figur 4 zeigt die beiden Konzentratleitungen 40, 50, von denen eine mit einen sauren Konzentrat, das sich in dem Behälter 42 befindet, und von denen die andere mit einem basischen Konzentrat, das sich in dem Behälter 52 befindet, in Fluidverbindung steht. In den Konzentratleitungen befinden sich stromabwärts der Behälter 42, 52 Filter F. Zwischen den jeweiligen Behältern 42, 52 und den Konzentratpumpen 41, 51, die die Konzentrate in die Mischkammer pumpen, befinden sich in jeder der Leitungen 40, 50 jeweils zwei Sensoren in Form von elektrisch leitfähigen Elementen S1 und S2 sowie S3 und S4.

Die nicht dargestellte Steuereinheit misst die Leitwerte zwischen den Sensoren S1 und S2 einerseits und zwischen den Sensoren S3 und S4 andererseits.

Wird dabei ein- oder mehrmals ein Leitwert gemessen, der unterhalb einer Schwelle liegt, wird ein Alarm ausgelöst und auf das Vorhandensein von Luft in der betreffenden Leitung 40, 50 geschlossen. Grundsätzlich ist von der Erfindung auch umfasst, dass der Leitwert etc. zwischen einem Sensor S1 oder S2 und S3 oder S4, d.h. über die Mischkammer hinweg gemessen wird.

Im Rahmen des normalen Betriebs, d.h. wenn keine Luft in den Konzentratleitungen festgestellt wird, wird die normale Luftabscheidung durchgeführt, wie sie oben beschrieben und in Figur 2 dargestellt ist. Diese Luftabscheidung erfolgt durch Öffnen des Ventils V und zwar zum Totzeitpunkt t1 der Bilanzkammer bzw. der mit frischer Dialyselösung gefüllten Bilanzkammerhälfte, anschließend während des Bilanzkammerzyklus BZ nicht mehr.

Wird allerdings durch die leitfähigen Elemente bzw. durch die mit diesen verbundene Steuereinheit erkannt, dass übermäßiger Lufteintrag stattfindet, was z.B. durch das Fehlen eines Konzentratbehälters 42, 52 oder dessen Leerzustand bedingt sein kann, wird dies von der Steuereinheit erfasst, die daraufhin eine oder mehrere Maßnahmen einleitet.

In einer Ausführungsform initiiert die Steuereinheit die folgenden Schritte:
Zunächst wird die Flussrate der Dialyselösung gegenüber dem normalen Dialysebetrieb erhöht, z.B. auf einen Wert von 500 ml/min.

Sodann werden die Bilanzkammerhälften durch das Öffnen der Ventile so verschaltet, dass ein Kurzschluss zwischen den jeweiligen Bilanzkammerhälften der zwei Bilanzkammern oder einer Bilanzkammer erfolgt, d.h. es erfolgt keine Förderung von Lösung in die Leitung L2.

Des Weiteren wird ein für den Nutzer wahrnehmbarer Alarm ausgelöst, der z.B. anzeigt, dass das saure Konzentrat oder das basische Konzentrat aufgebraucht ist.

Sodann wird von dem normalen Luftabscheidemodus gemäß Figur 2 auf die Spezialluftabscheidung gemäß Figur 3 umgeschaltet. Diese zeichnet sich dadurch aus, dass in der Hochdruckphase (T3) der mit frischer Dialyselösung gefüllten Bilanzkammerhälfte das Ventil V gemäß Figur 1 einmal oder vorzugsweise mehrfach, z.B. dreimal geöffnet wird. In der Hochdruckphase der Bilanzkammerhälfte ist diese vollständig mit Flüssigkeit gefüllt. Dies hat zur Folge, dass das Öffnen des Ventils V nicht zu einer Strömung von mit Luft angereicherter Flüssigkeit in die Bilanzkammer führt. Vielmehr wird durch das Öffnen und Schließen des Ventils Luft aus der Mischkammer 20 entfernt, so dass bei dem Zurückschalten in den normalen Betriebsmodus bzw. normalen Luftabscheidemodus keine oder nur wenig Luft in die Bilanzkammer gelangt. Dadurch lässt sich die Wahrscheinlichkeit für das Auftreten von Fehlbilanzierungen vermeiden oder zumindest verringern.

Figur 3 zeigt den Verlauf des Druckes über die Zeit in einer Anordnung gemäß Figur 2, wobei gleiche Zeitpunkte und Zeitspannen mit denselben Bezugszeichen versehen sind, und verdeutlicht, dass in der Hochdruckphase T3 das Ventil V dreimal geöffnet und geschlossen wird, was durch das Bezugszeichen V' gekennzeichnet ist und sich durch entsprechende Druckschwankungen bemerkbar macht. Ein Öffnen und Schließen des Ventils V zum Totzeitpunkt t1 findet vorzugsweise nicht statt.

Die in Figur 2 und 3 angegebenen Druckwerte sowie sämtliche andere im Rahmen der vorliegenden Erfindung genannten Werte sind exemplarischer Natur und nicht beschränkend.

Wird festgestellt, dass in den Konzentratleitungen wieder Konzentrat vorliegt, wird von der Spezialluftabscheidung gemäß Figur 3 wieder auf die normale Luftabscheidung gemäß Figur 2 umgeschaltet.

Ist die Konzentratversorgung wieder hergestellt, indem gefüllte Konzentratbehälter wieder an die Konzentratleitungen angeschlossen sind, und wurde für einen bestimmten Zeitraum oder nach Ablauf von z.B. 5 Bilanzkammerzyklen keine Luft mehr in den Konzentratleitungen erfasst, wird der Spülvorgang eingeleitet.

Beim Spülvorgang wird die Hydraulik, d.h. der Dialyselösungskreislauf oder ein Teil von diesem durchspült. Dieser Bereich kann Luftabscheider des Dialyselösungskreislaufes, Filter, die Bilanzkammer, Ventile und alle Leitungen umfassen, die die vorgenannten Elemente verbinden. Der Spülvorgang wird beispielsweise so durchgeführt, dass der Hydraulikkreislauf oder ein Teil von diesem zwei Mal mit gebrauchsfertiger Dialyselösung durchströmt wird.

Vorzugsweise ist der Patient während des Spülvorgangs von dem Dialysegerät getrennt.

Ist der Spülvorgang abgeschlossen, wird die Alarmmeldung gelöscht, der Dialysatfluss wieder auf den Sollwert verringert und der Patient wieder angeschlossen, so dass die Behandlung fortgeführt werden kann.

## Patentansprüche

1. Dialysegerät, das ausgebildet ist, mit einem extrakorporalen Blutsystem ausgerüstet zu werden, mit einem Dialyselösungssystem, das ausgebildet ist, mit einem Dialysator ausgerüstet zu werden, wobei ein Mischbereich, insbesondere eine Mischkammer (20), vorgesehen ist, in dem sich im Betrieb des Dialysegerätes die frische Dialyselösung oder ein Bestandteil von dieser befindet, wobei mit dem Mischbereich eine Konzentratleitung (40, 50) in Fluidverbindung steht, durch die Konzentrat aus einem Konzentratbehälter (42, 52) in den Mischbereich einleitbar ist, wobei in oder an der oder den Konzentratleitungen (40, 50) voneinander beabstandet zumindest zwei elektrisch leitfähige Elemente (S1, S2, S3, S4) angeordnet sind, dass eine Messeinrichtung vorgesehen ist, die mit den leitfähigen Elementen (S1, S2, S3, S4) in elektrischer Verbindung steht und die ausgebildet ist, zwischen diesen den Leitwert oder eine damit korrelierte Größe zu messen, und dass eine Steuereinheit vorgesehen ist, die ausgebildet ist, den gemessenen Wert mit einem Sollwert, Grenzwert oder Sollwertbereich zu vergleichen, wobei die Steuereinheit ausgebildet ist, eine besondere Betriebsweise des Dialysegerätes zu initiieren, wenn der gemessene Wert nicht mit dem Sollwert übereinstimmt, einen Grenzwert über- oder unterschreitet oder nicht in einem Sollwertbereich liegt, wobei die Steuereinheit derart ausgebildet ist, dass die besondere Betriebsweise eine vom Normalbetrieb abweichende Spezialluftabscheidung und die Spülung des Dialyselösungssystems oder eines Teils von diesem umfasst, **dadurch gekennzeichnet, dass** die Steuereinheit derart ausgebildet ist, dass die Spülung des Dialyselösungssystems oder eines Teils von diesem erst nach der Spezialluftabscheidung erfolgt.

2. Dialysegerät nach Anspruch 1, **dadurch gekennzeichnet, dass** in oder an der Konzentratleitung (40, 50) jeweils genau ein elektrisch leitfähiges Element (S1, S2, S3, S4) angeordnet ist und/oder dass eine oder mehrere Konzentratleitungen (40, 50) vorgesehen sind, in oder an der jeweils mehrere elektrisch leitfähige Elemente (S1, S2, S3, S4) angeornet sind.

3. Dialysegerät nach Anspruch 1 oder 2, **dadurch gekennzeichnet, dass** die leitfähigen Elemente (S1, S2, S3, S4) durch Schlauchtüllen gebildet werden und/oder dass die leitfähigen Elemente (S1, S2, S3, S4) aus Metall, vorzugsweise aus Edelstahl und/oder Titan bestehen und/oder dass die leitfähigen Elemente (S1, S2, S3, S4) in oder an derselben Konzentratleitung (40, 50) oder in oder an verschiedenen Konzentratleitungen (40, 50) angeordnet sind.

4. Dialysegerät nach einem der vorhergehenden Ansprüche, **dadurch gekennzeichnet, dass** sich in der Konzentratleitung (40, 50) eine Konzentratpumpe (41, 51) befindet und dass sich die leitfähigen Elemente (S1, S2, S3, S4) in Strömungsrichtung des Konzentrats stromaufwärts oder stromabwärts der Konzentratpumpe (41, 51) befinden.

5. Dialysegerät nach einem der vorhergehenden Ansprüche, **dadurch gekennzeichnet, dass** die Steuereinheit ausgebildet ist, einen Alarm zu initiieren, wenn der gemessene Wert nicht mit dem Sollwert übereinstimmt, einen Grenzwert über- oder unterschreitet oder nicht in einem Sollwertbereich liegt.

6. Dialysegerät nach Anspruch 1 oder Anspruch 5, **dadurch gekennzeichnet, dass** die Steuereinheit ausgebildet ist, dass der Alarm und/oder die besondere Betriebsweise des Dialysegerätes erst dann initiiert wird, wenn der gemessene Wert häufiger als ein Schwellwert nicht mit dem Sollwert übereinstimmt, einen Grenzwert über- oder unterschreitet oder oder nicht in einem Sollwertbereich liegt.

7. Dialysegerät nach einem der Ansprüche 1, 5 oder 6, **dadurch gekennzeichnet, dass** die Steuereinheit derart ausgebildet ist, dass die besondere Betriebsweise die Erhöhung der Flussrate der Dialyselösung und/oder die Bypassschaltung eines in dem Dialyselösungssystem angeordneten Dialysators umfasst.

8. Dialysegerät nach einem der vorhergehenden Ansprüche, **dadurch gekennzeichnet, dass** sich in der Konzentratleitung (40, 51) kein Luftabscheidemittel, insbesondere keine Luftabscheidekammer befindet und/oder zwischen der Mündung der Konzentratleitung (40, 50) in den Mischbereich und einer Bilanzkammer des Dialyselösungssystems kein Lufabscheidemittel befindet.

9. Dialysegerät nach einem der Ansprüche 7 und 8, **dadurch gekennzeichnet, dass** die Steuereinheit derart ausgebildet ist, dass diese in dem Betriebszustand der Spezialluftabscheidung des Dialysegerätes ein vorzugsweise mit dem Mischbereich in Fluidverbindung stehendes Ventil ein- oder mehrmals öffnet und schließt, wenn sich die mit der genannten Leitung in Fluidverbindung stehende Bilanzkammerhälfte einer Bilanzkammer des Dialyselösungssystems in ihrer Hochdruckphase befindet.

10. Dialysegerät nach einem der vorhergehenden Ansprüche, **dadurch gekennzeichnet, dass** der Mischbereich, insbesondere die Mischkammer (20) mit einem Frischwasseranschluss, insbesondere mit einem RO-Wasseranschluss in Verbindung steht oder mit diesem verbindbar ist.

## Claims

1. Dialysis machine, which is configured to be equipped with an extracorporeal blood system, comprising a dialysis solution system which is configured to be equipped with a dialyzer, wherein a mixing region, in particular a mixing chamber (20), is provided in which the fresh dialysis solution or a component thereof is located in the operation of the dialysis machine, wherein a concentrate line (40, 50) is in fluid communication with the mixing region, through which concentrate line concentrate can be conducted from a concentrate container (42, 52) into the mixing region, wherein at least two electrically conductive elements (S1, S2, S3, S4) are arranged spaced apart from one another in or at the concentrate line or lines (40, 50), a measuring device is provided which is electrically connected to the conductive elements (S1, S2, S3, S4) and which is configured to measure the conductance or a value correlated therewith between them, and a control unit is provided which is configured to compare the measured value with a desired value, a limit value, or a desired value range, wherein the control unit is configured to initiate a special operating mode of the dialysis machine when the measured value does not agree with the desired value, exceeds or falls below a limit value, or is not in a desired value range, wherein the control unit is configured such that the special operating mode comprises a special air separation differing from normal operation and/or the flushing of the dialysis solution system or a part thereof, **characterized in that** the control unit is configured such that the flushing of the dialysis solution system or a part thereof only takes place after the special air separation.

2. Dialysis machine in accordance with claim 1, **characterized in that** exactly one electrically conductive element (S1, S2, S3, S4) is respectively arranged in or at the concentrate line (40, 50) and/or that one or more concentrate lines (40, 50) are provided in or at which a plurality of electrically conductive elements (S1, S2, S3, S4) are arranged.

3. Dialysis machine in accordance with claim 1 or claim 2, **characterized in that** the conductive elements (S1, S2, S3, S4) are formed by hose connections, and/or **in that** the conductive elements (S1, S2, S3, S4) consist of metal, preferably of stainless steel and/or titanium, and/or **in that** the conductive elements (S1, S2, S3, S4) are arranged in or at the same concentrate line (40, 50) or in or at different concentrate lines (40, 50).

4. Dialysis machine in accordance with any one of the preceding claims, **characterized in that** a concentrate pump (41, 51) is located in the concentrate line (40), and **in that** the conductive elements (S1, S2, S3, S4) are located upstream or downstream of the concentrate pump (41, 51) in the direction of flow of the concentrate.

5. Dialysis machine in accordance with any one of the preceding claims, **characterized in that** the control unit is configured to initiate an alarm and/or a special operating mode of the dialysis machine when the measured value does not agree with the desired value, exceeds or falls below a limit value, or is not in a desired value range.

6. Dialysis machine in accordance with claim 1 or claim 5, **characterized in that** the control unit is configured such that the alarm and/or the special operating mode of the dialysis machine is only initiated when the measured value does not agree with the desired value, exceeds or falls below a limit value, or is not in a desired value range in each case more frequently than a threshold value.

7. Dialysis machine in accordance with any one of the claims 1, 5 or 6, **characterized in that** the control unit is configured such that the special operating mode comprises the increase of the flow rate of the dialysis solution and/or the bypass circuit of a dialyzer arranged in the dialysis solution system.

8. Dialysis machine in accordance with any one of the preceding claims, **characterized in that** no air separation means, in particular no air separation chamber, is located in the concentrate line (40, 51) and/or no air separation means is located between the opening of the concentrate line (40, 50) into the mixing region and a balancing chamber of the dialysis solution system.

9. Dialysis machine in accordance with any one of the claims 7 and 8, **characterized in that** the control unit is configured such that it opens and closes a valve that is preferably in fluid communication with the mixing region once or multiple times in the operating state of the special air separation of the dialysis machine when the balancing chamber half of a balancing chamber of the dialysis solution system in fluid communication with said line is in its high pressure phase.

10. Dialysis machine in accordance with any one of the preceding claims, **characterized in that** the mixing region, in particular the mixing chamber (20), is connected or connectable to a fresh water connector, in particular to an RO water connector.

## Revendications

1. Appareil de dialyse, qui est configuré pour être équipé d'un système sanguin extracorporel, avec un système de solution de dialyse, qui est configuré pour être équipé d'un dialyseur, une zone de mélange, notamment une chambre de mélange (20), étant prévue, dans laquelle se trouve, pendant le fonctionnement de l'appareil de dialyse, la solution de dialyse fraîche ou un constituant de celle-ci, une conduite de concentré (40, 50) étant en communication fluidique avec la chambre de mélange, par laquelle du concentré provenant d'un réservoir de concentré (42, 52) peut être introduit dans la zone de mélange, au moins deux éléments électriquement conducteurs (S1, S2, S3, S4) étant agencés à distance l'un de l'autre dans ou sur la ou les conduites de concentré (40, 50), il étant prévu un dispositif de mesure, qui est en liaison électrique avec les éléments conducteurs (S1, S2, S3, S4) et qui est configuré pour mesurer entre ceux-ci la valeur de conduction ou une grandeur qui lui est corrélée, et il étant prévu une unité de commande, qui est configurée pour comparer la valeur mesurée à une valeur de consigne, une valeur limite ou une plage de valeurs de consigne, l'unité de commande étant configurée pour déclencher un mode de fonctionnement particulier de l'appareil de dialyse lorsque la valeur mesurée ne correspond pas à la valeur de consigne, est supérieure ou inférieure à une valeur limite ou ne se situe pas dans une plage de valeurs de consigne, l'unité de commande étant configurée de telle sorte que le mode de fonctionnement particulier comprend une séparation d'air spéciale différente du fonctionnement normal et le rinçage du système de solution de dialyse ou d'une partie de celui-ci, **caractérisé en ce que** l'unité de commande est configurée de telle sorte que le rinçage du système de solution de dialyse ou d'une partie de celui-ci n'a lieu qu'après la séparation d'air spéciale.

2. Appareil de dialyse selon la revendication 1, **caractérisé en ce que** dans ou sur la conduite de concentré (40, 50) est agencé respectivement exactement un élément électriquement conducteur (S1, S2, S3, S4) et/ou **en ce qu'**il est prévu une ou plusieurs conduites de concentré (40, 50) dans ou sur lesquelles sont agencés respectivement plusieurs éléments électriquement conducteurs (S1, S2, S3, S4).

3. Appareil de dialyse selon la revendication 1 ou 2, **caractérisé en ce que** les éléments conducteurs (S1, S2, S3, S4) sont formés par des manchons de tuyau et/ou **en ce que** les éléments conducteurs (S1, S2, S3, S4) sont constitués de métal, de préférence d'acier inoxydable et/ou de titane et/ou **en ce que** les éléments conducteurs (S1, S2, S3, S4) sont agencés dans ou sur la même conduite de concentré (40, 50) ou dans ou sur différentes conduites de concentré (40, 50).

4. Appareil de dialyse selon l'une quelconque des revendications précédentes, **caractérisé en ce qu'**une pompe à concentré (41, 51) se trouve dans la conduite de concentré (40, 50) et **en ce que** les éléments conducteurs (S1, S2, S3, S4) se trouvent en amont ou en aval de la pompe à concentré (41, 51) dans la direction d'écoulement du concentré.

5. Appareil de dialyse selon l'une quelconque des revendications précédentes, **caractérisé en ce que** l'unité de commande est configurée pour déclencher une alarme lorsque la valeur mesurée ne correspond pas à la valeur de consigne, est supérieure ou inférieure à une valeur limite ou ne se situe pas dans une plage de valeurs de consigne.

6. Appareil de dialyse selon la revendication 1 ou la revendication 5, **caractérisé en ce que** l'unité de commande est configurée de telle sorte que l'alarme et/ou le mode de fonctionnement particulier de l'appareil de dialyse ne soit déclenché que lorsque la valeur mesurée ne correspond pas à la valeur de consigne, est supérieure ou inférieure à une valeur limite ou ne se trouve pas dans une plage de valeurs de consigne, et ce plus fréquemment qu'une valeur seuil.

7. Appareil de dialyse selon l'une quelconque des revendications 1, 5 ou 6, **caractérisé en ce que** l'unité de commande est configurée de telle sorte que le mode de fonctionnement particulier comprend l'augmentation du débit de la solution de dialyse et/ou la mise en dérivation d'un dialyseur agencé dans le système de solution de dialyse.

8. Appareil de dialyse selon l'une quelconque des revendications précédentes, **caractérisé en ce qu'**aucun moyen de séparation d'air, notamment aucune chambre de séparation d'air, ne se trouve dans la conduite de concentré (40, 51) et/ou aucun moyen de séparation d'air ne se trouve entre l'embouchure de la conduite de concentré (40, 50) dans la zone de mélange et une chambre d'équilibrage du système de solution de dialyse.

9. Appareil de dialyse selon l'une quelconque des revendications 7 et 8, **caractérisé en ce que** l'unité de commande est configurée de telle sorte que, dans le mode de fonctionnement de la séparation d'air spéciale de l'appareil de dialyse, elle ouvre et ferme une ou plusieurs fois une soupape, de préférence en communication fluidique avec la zone de mélange, lorsque la moitié de la chambre d'équilibrage d'une chambre d'équilibrage du système de solution de dialyse, en communication fluidique avec ladite conduite, se trouve dans sa phase de haute pression.

10. Appareil de dialyse selon l'une quelconque des revendications précédentes, **caractérisé en ce que** la zone de mélange, notamment la chambre de mélange (20), est reliée ou peut être reliée à un raccord d'eau fraîche, notamment à un raccord d'eau OI.
